(19) Europäisches Patentamt
European Patent Office
Office européen des brevets

(11) EP 0 427 168 B1

(12) EUROPEAN PATENT SPECIFICATION

(45) Date of publication and mention of the grant of the patent:
**13.03.1996 Bulletin 1996/11**

(51) Int Cl.[6]: **C07D 249/08**, A01N 43/653

(21) Application number: **90121117.7**

(22) Date of filing: **14.12.1981**

(54) **Optical isomer of triazolylpentenols, and their production and use as fungicide, herbicide and/or plant growth regulant**

Optisch isomeres Triazolylpentenol. Verfahren zur Herstellung und Verwendung als Fungizid, Herbizid und/oder Wachstumsregulator

Isomère optique de triazolylpenténols, leur préparation et usage comme fongicide, herbicide et/ou régulateur de croissance

(84) Designated Contracting States:
**BE CH DE FR GB IT LI NL**

(30) Priority: **15.12.1980 JP 177704/80**
**22.12.1980 JP 182407/80**

(43) Date of publication of application:
**15.05.1991 Bulletin 1991/20**

(62) Application number of earlier application in accordance with Art. 76 EPC: **84200456.6**

(73) Proprietor: **SUMITOMO CHEMICAL COMPANY, LIMITED**
**Osaka (JP)**

(72) Inventors:
- **Funaki, Yuji**
  **Toyonaka-shi (JP)**
- **Yoneyoshi, Yukio**
  **Otsu-shi (JP)**
- **Oguri, Yukio**
  **Toyonaka-shi (JP)**
- **Izumi, Kazuo**
  **Nishinomiya-shi (JP)**

(74) Representative:
**Cresswell, Thomas Anthony et al**
**J.A. KEMP & CO.**
**14 South Square**
**Gray's Inn**
**London WC1R 5LX (GB)**

(56) References cited:
**EP-A- 0 015 639**
**DE-A- 2 920 437**
**GB-A- 2 046 260**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 427 168 B1

## Description

This invention relates to an optically active triazolyl alcohol derivative having an optical activity of (+) and E-geometric configuration, and which may be represented by the formula (I),

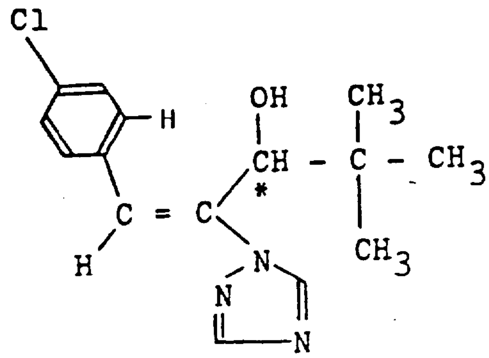

(I)

wherein the asterisk indicates an asymmetric carbon atom, salts thereof, a process for preparing the same, plant growth regulatory and herbicidal compositions containing the same as an active ingredient and the use of this compound in the preparation of plant growth regulatory and herbicidal compositions.

The present application is divided from European Patent Application No. 813058732.2, granted as EP 054 431.

The racemic triazolyl alcohol derivatives and their excellent fungicidal, plant growth regulatory, and herbicidal activities have already been described in Japanese Patent Application "Kokai" (Laid-open) No. 124,771/1980 and Japanese Patent Application No. 100,547/1980 as well as in DE-A 2 290 437 and GB-A-2 046 460.

The triazolyl alcohol derivative of formula (I) has optical isomers due to the asymmetric carbon atom (*C). The triazolyl alcohol derivative (I) having an optical activity of (+), as referred to above, is an optical isomer which shows an optical rotation of (+), as measured in chloroform with sodium D line, and is hereinafter referred to as the (+)-triazolyl alcohol derivative. On the other hand, another isomer which shows an optical rotation of (-) is hereinafter referred to as (-)-triazolyl alcohol derivative. Salts of the triazolyl alcohol derivative are also included in the scope of this invention. These salts are salts with plant-physiologically tolerable acids. Examples of such acids include hydrogen halides such as hydrobromic acid, hydrochloric acid and hydroiodic acid; carboxylic acids such as acetic acid, trichloroacetic acid, maleic acid and succinic acid; sulfonic acids such as p-toluenesulfonic acid and methanesulfonic acid; nitric acid, sulfuric acid, and phosphoric acid. These salts are obtained in a customary manner.

The present inventors examined in detail the usefulness of (-)- or (+)-triazolyl alcohol derivatives (I) obtained by the process of this invention. Upon comparison of (-)-, (+)- and racemic triazolyl alcohol derivatives with one another, it was found that fungicidal activity falls in the order : (-)-triazolyl alcohol derivative > racemic triazolyl alcohol derivative > (+) -triazolyl alcohol derivative, whilst plant growth regulatory activity and herbicidal activity are in the order : (+)-triazolyl alcohol derivative > racemic triazolylalcohol derivative > (-)-triazolyl alcohol derivative. In short, the present inventors have discovered an entirely new fact that a (-)-triazolyl alcohol derivative exhibits an excellent fungicidal activity, while a (+)-triazolyl alcohol derivative exhibits an excellent plant growth regulatory activity and herbicidal activity.

The present invention contributes much to the cultivation of plants in the fields of agriculture and horticulture. For instance, application of a more active chemical is connected to adequate application of a smaller amount of the chemical, which leads to the improvement in economics of the proesses of manufacture, transportation and field application and to the expectation of minimizing the environmental pollution as well as the improvement in safety.

As stated above, the (+)-triazolyl alcohol derivative can be utilized as a plant growth regulator to regulate the growth of useful plants. For instance, they can be applied to keep rice plants, wheat and barley, lawn grass, hedge plants and fruit trees from spindle growth and also to effect dwarfing of potted garden plants such as chrysanthemum, pansy, poinsettia, azalea and rhododendron. In rice cropping and wheat or barley cropping, the lodging of rice, wheat or barley plants caused by excessive application of fertilizers or by gales often presents an important problem. By applying the (+)-triazolyl alcohol derivative to rice, wheat or barley at a proper stage of growth, spindling can be suppressed so that the plant height may be suitably controlled to keep effectively the plant from lodging. In the cultivation of chrysanthemum in pot, application of the compound results in a reduction in stem length without injurious effect on the flower, thus improving the commercial value of the plant.

Further, the (+)-triazolyl alcohol derivative exhibits a strong herbicidal activity against gramineous weeds such as barnyard millet, large crabgrass and green foxtail; cyperaceous weeds such as purple nutsedge; broad-leaved weeds in upland fields such as green amaranth, fat hen, common purslane and common chickweed; annual and perennial weeds in paddy fields such as barnyardgrass, monochoria, spike-flowered rotala, Dapatrium junceum, bulrush and slender spikerush.

When applied to an upland field, the (+)triazolyl alcohol derivative exhibits a strong activity against principal weeds and is effective for the pre-emergence treatment of soil as well as for foliage treatment in an early stage of growth. The compound has tremendous advantages in that it has no harmful effect on principal crops such as rice, soybean, cotton, corn, peanut, sunflower and sugar beet and can be safely used also for vegetables such as lettuce, radish and tomato. The compound, therefore, is useful for weeding in a variety of grain fields, vegetable gardens, orchards, lawns, pastures, tea fields, mulberry fields, rubber plantations, forest lands, and non-cultivation fields. It was found, moreover, that the compound is highly nontoxic to mammals and fish and is substantially harmless to agriculturally useful crops.

Methods for preparing the (+)-triazolyl alcohol derivatives include those used in preparing conventional optically active substances such as asymmetric reduction and resolution of the diastereomer obtained from a racemate and an optically active reactive compound. These methods are described below in detail.

(1) Preparation by asymmetric reduction.

The racemate of the present compound is obtained by reducing a ketone compound represented by the general formula (II) with a metal-hydrogen complex such as lithium aluminum hydride ($LiAlH_4$) or sodium borohydride ($NaBH_4$) [Japanese Patent Publication "Kokai" (Laid-open) No. 124,771/1980].

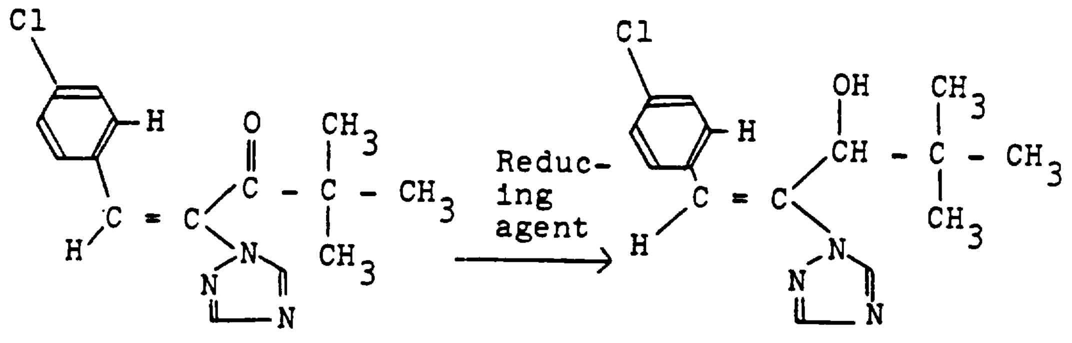


(II) (I) Racemate

Asymmetric reduction is commonly conducted by utilizing the enantioselective reaction which takes place when a ketone compound (II) is reduced with a chiral metal-hydrogen complex. A few such procedures are described below.

(a) It is a general practice to use as the chiral metal-hydrogen complex a reducing agent of the chiral modified lithium aluminum hydride type formed by the partial decomposition of lithium aluminum hydride with an optically active alcohol [Literature: Tetrahedron Letters, Vol. 29, 913 (1973); Bull. Soc. Chim. Fr., 1968, 3795; J. Org. Chem., 38 (10), 1973; Tetrahedron Letters, Vol. 36, 3165 (1976)].

Among examples of the optically active alcohols used in this invention as asymmetric source, may be cited (-) -menthol, (-)-borneol, (-)-N-methylephedrine, and (-)-2-N,N-dimethylamino-1-phenylethanol. It is of course possible to use the (-) optically active form of other optically active alcohols including alkaloids, carbohydrates and amino alcohols such as, for example, quinine, cis-Myrtanol, 2-N-benzyl-N-methylamino-1-phenylethanol and 4-dimethylamino-3-methyl-1,2-diphenyl-2-butanol. The formation of a chiral modified lithium aluminum hydride reducing agent using an optically active alcohol as asymmetric source can be effected by adding 1 to 3 equivalent ratio of the optically active alcohol to one equivalent ratio of lithium aluminum hydride suspended in a suitable solvent. It is a general practice to use as the solvent an ether such as diethyl ether, tetrahydrofuran or dioxane, though an aromatic hydrocarbon such as benzene or toluene or an aliphatic hydrocarbon such as n-hexane or n-pentane may also be used.

(b) It is sometimes advantageous to use as the chiral metal-hydrogen complex a chiral modified lithium aluminum hydride reducing agent formed by the reaction of one equivalent ratio of an optically active alcohol, 2 equivalent ratio of a N-substituted aniline represented by the general formula (III),

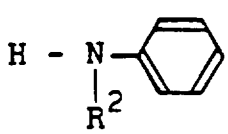

(III)

wherein $R^2$ is a lower alkyl group or a phenyl group, and one equivalent ratio of lithium aluminum hydride[literature: Tetrahedron Letters, Vol. 21, 2753 (1980)]. The optically active alcohol used as asymmetric source in this invention is exemplified by the (-) optically active forms of an optically active amino alcohol such as N-substituted ephedrine, for

example (-)-N-methylephedrine and 2-N,N-disubstituted-amino-1-phenylethanol for example (-)-2-N,N-dimethylamino-1-phenylethanol. As to the N-substituted aniline, a desirable result is obtained by use of a lower alkylsubstituted aniline such as N-methylaniline or N-ethylaniline, or diphenylamine. The preparation of such a chiral modified lithium aluminum hydride reducing agent can be achieved by suspending one equivalent ratio of lithium aluminum hydride ($LiAlH_4$) in a suitable solvent and admixing with one equivalent ratio of an optically active alcohol followed by 2 equivalent ratio of a N-substituted aniline. The solvent described above in (a) can be used likewise.

The asymmetric reduction is conducted by adding a ketone compound (II) dissolved in a suitable solvent to the chiral modified lithium aluminum hydride prepared as in (a) or (b) described above. The solvent is the same as described in (a). The reaction temperature is preferably 0°C or below, though a temperature between -80°C and the boiling point of the solvent can be used. After completion of the reaction, the complex compound is decomposed by the addition of a dilute aqueous acidic solution and the reaction mixture is purified by extraction, silica gel column chromatography or recrystallization to obtain the intended product.

(2) Preparation by resolution of diastereomers.

A method for resolving optical isomers by use of diastereomer esters formed from a racemic alcohol compound and an optically active reactive compound has been known (literature: Org. Reaction, Vol. 2, 380). A diastereomeric ester mixture (IV) is obtained by allowing a racemate of triazolyl alcohol compound (I) to react with a reactive derivative of optically active carboxylic acid in the presence of a base. The (+)-triazolyl alcohol derivative (I) is obtained by resolving said diastereomeric ester mixture by chromatography or fractional crystallization into the ester of the (+)-triazolyl alcohol and the ester of the (-)-triazolyl alcohol, and decomposing the ester of the (+)-triazolyl alcohol.

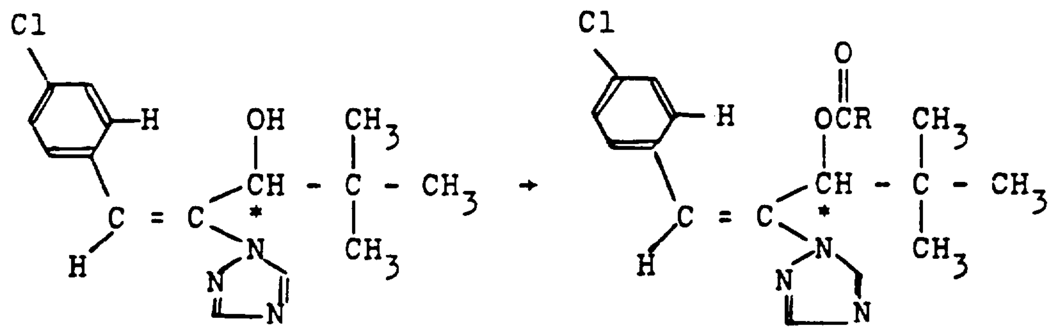

(I) Racemate (IV) Diastereomeric mixture

Resolution
→ (+)-Triazolyl alcohol ester → (+)-Triazolyl alcohol compound
→ (-)-Triazolyl alcohol ester → (-)-Triazolyl alcohol compound

(In the above formulae, the asterisk is defined as above.)

As examples of the optically active carboxylic acids for use in the esterification of racemate of a triazolyl alcohol (I), there are (-)-menthoxyacetic acid, (+)- or (-)-N-trifluoroacetylproline, (+)-camphoric acid, (+)- or (-)-mandelic acid, (+)- or (-)-2-phenylpropionic acid, (+)- or (-)-2-isopropyl-4'-chlorophenylacetic acid, (+)- or (-)-α-methoxy-α-trifluoromethylphenylacetic acid, (+)- or (-)-cis-chrysanthemic acid, and (+)- or (-)-transchrysanthemic acid. The reactive derivatives of these optically active carboxylic acids include acid halides and acid anhydrides. Generally, the optically active carboxylic acid is converted into an acid halide in a customary manner and allowed to react with the racemate of the triazolyl alcohol (I) to effect esterification. The reaction is conducted in a common inert solvent (e.g., acetone, acetonitrile, tetrahydrofuran, ethyl acetate, benzene, toluene, dichloromethane, chloroform and carbon tetrachloride) and in the presence of a dehydrohalogenation agent (e.g., triethylamine, N,N-dimethylaniline and pyridine). Generally, 1 to 5 moles of an acyl halide and a dehydrohalogenation agent are used for one mole of the triazolyl alcohol racemate (I). Pyridine

behaves also as a solvent when used in excess. The reaction temperature is in the range of from room temperature to the boiling point of the solvent. It is of course possible to prepare the diastereomeric ester by using the anhydride of an optically active carboxylic acid.

When the diastereomeric mixture of a triazolyl alcohol ester (V) obtained as described above is crystallizable, it is resolved by repeated fractional crystallization, while if it is in oily form, the resolution is effected by column chromatography or high-speed liquid chromatography. The ester of the (+)-triazolyl alcohol thus formed is decomposed in the presence of a base such as sodium hydroxide or potassium hydroxide in a suitable solvent such as water or an aqueous organic solvent (ethanol or methanol is generally used) to obtain the (+)-triazolyl alcohol derivative (I).

In field application of the compound of this invention obtained as described above, it may be used either alone without the addition of other ingredients or in mixtures with a carrier to make it more convenient for use as a herbicide and a plant growth regulator. Examples of the usual preparation forms include dust, wettable powder, oil spray, emulsion, tablet, granule, fine granule, aerosol and flowable. These preparations contain generally 0.1 to 95.0%, preferably 0.2 to 90.0% by weight of the active compound (including other active ingredients). A suitable application rate is 2 to 500 g/10 ares and a preferable concentration of the active ingredients for field application is 0.001 to 1.0%. However, the concentration may be suitably increased or decreased without sticking to the said range, because the application rate and the concentration depend on the type of preparation, application time of the year, method of application, site of application and the type of crop to be treated.

For use as a herbicide and plant growth regulator, the present compound can be mixed with fungicides and insecticides, and a synergistic effect is expectable from such a mixture. Examples of fungicides include N-(3,5-dichlorophenyl) -1,2-dimethylcyclopropane-1,2-dicarboximide, S-n-butyl S-p-tert-butylbenzyldithiocarbonimidate, 0,0-dimethyl 0-(2,6-dichloro-4-methylphenyl)phosphorothioate, methyl 1-butylcarbamoyl-1H-benzimidazol-2-yl-carbamate, N-trichloromethylthio-4-cyclohexene-1,2-dicarboximide, cis-N-(1,1,2,2-tetrachloroethylthio)-4-cyclohexene-1,2-dicarboximide, Polyoxin, streptomycin, zinc ethylenebisdithiocarbamate, zinc dimethylthiocarbamate, manganese ethylenebisdithiocarbamate, bis(N,N-dimethylthiocarbamoyl)disulfide, tetrachloroisophthalonitrile, 8-hydroxyquinoline, dodecylguanidine acetate, 5,6-dihydro-2-methyl-1,4-oxathiin-5-carboxanilide, N'-dichlorofluoromethylthio-N,N-dimethyl-N'-phenylsulfamide, 1-(4-chlorophenoxy)-3,3-dimethyl-1-(1,2,4-triazol-1-yl)-2-butanone, 1,2-bis(3-methoxycarbonyl-2-thioureid)benzene, methyl N-(2,6-dimethylphenyl)-N-methoxyacetyl-2-methylglycinate, and aluminum ethylphosphite. Examples of the insecticides include organophosphorus insecticides such as 0,0-dimethyl O-(4-nitro-3-methylphenyl)phosphorothioate, O-(4-cyanophenyl) 0,0-dimethylphosphorothioate, O-(4-cyanophenyl) O-ethylphenylphosphonothioate, 0,0-dimethyl S-(1-ethoxycarbamoylmethyl)phosphorodithioate, 2-methoxy-4H-1,3,2-benzodioxaphosphorin-2-sulfide, 0,0-dimethyl S-(1-ethoxycarbonyl-1-phenylmethyl)phosphorodithioate, and pyrethroid insecticides such as α-cyano-3-phenoxybenzyl 2-(4-chlorophenyl)isovalerate, 3-phenoxybenzyl 2,2-dimethyl-3-(2,2-dichlorovinyl)cyclopropanecarboxylate, and α-cyano-3-phenoxybenzyl 2',2'-dimethyl-3'-(2,2-dibromovinyl)cyclopropanecarboxylate.

The invention is further illustrated below in detail with reference to Examples, Reference Examples, Test Examples and Formulation Examples. The Formulation Examples show typical formulations of triazolyl alcohols such as the compound of the invention. Examples of further reagents which may be used in producing the present compound can be seen in EP-A-0 054 431.

Example 1

Synthesis of (+)-(E)-1-(4-chlorophenyl)-2-(1,2,4-triazol-1-yl)-4,4-dimethyl-1-penten-3-ol by resolution of diastereomeric ester:

A mixture of 4.3 g of (±)-(E)-1-(4-chlorophenyl)-2-(1,2,4-triazol-1-yl)-4,4-dimethyl-1-penten-3-ol and 8 g of (-)-menthoxyacetyl chloride was stirred in 50 cc of pyridine at 70°C for 7 hours. The reaction mixture was poured into 200 cc of ice water and extracted with 400 cc of ethyl acetate. The organic layer was washed successively with 0.5N hydrochloric acid, 200 cc of a saturated aqueous sodium hydrogencarbonate solution and 200 cc of ice-cooled water, then dried over anhydrous sodium sulfate, and concentrated in vacuo. The resulting oily crude substance was purified by silica gel column chromatography (150 g of silica gel; developing solvent; n-hexane/acetone = 30 : 1) to obtain 7.4 g of (+)-[(E) -1-(4-chlorphenyl)-2-(1,2,4-triazol-1-yl)-4,4-dimethyl-1-penten-3-yl]-(-)-menthoxy acetate. Upon repetition of chromatography on another silica gel column (250 g of silica gel; developing solvent; n-hexane/benzene/acetone = 20/20/1), there were obtained 2.6 g of (-)-[(E)-1-(4-chlorophenyl)-2-(1,2,4-triazol-1-yl)-4,4-dimethyl-1-penten-3-yl]-(-)-menthoxyacetate ($n_D^{25}$ 1.5265) as first eluate, 3 g of the diastereomeric ester mixture as second eluate, and 1.2 g of (+)-[(E)-1-(4-chlorophenyl)-2-(1,2,4-triazol-1-yl)-4,4-dimethyl-1-penten-3-yl]-(-)-menthoxyacetate ($n_D^{25}$ 1.5281) as final eluate.

1.2 g of (+)-[(E)-1-(4-chlorophenyl)-2-(1,2,4-triazol-1-yl)-4,4-dimethyl-1-penten-3-yl]-(-)menthoxyacetate was treated with 20 cc of a 95% aqueous ethanol solution containing 0.2 g of potassium hydroxide and the resulting crude crystals

were recrystallized from a carbon tetrachloride-n-hexane mixture to obtain 0.5 g of (+)-(E)-1-(4-chlorophenyl)-2-(1,2,4-triazol-1-yl)-4,4-dimethyl-1-penten-3-ol: $[\alpha]_D^{24}$ + 14.0 (c = 1.0, $CHCl_3$); m.p. 169 - 170°C.

Reference Example 1

Synthesis of racemate of (E)-1-(4-chlorophenyl)-2-(1,2,4-triazol-1-yl)-4,4-dimethyl-1-penten-3-ol:

In 50 ml of methanol, was dissolved 2.9 g (0.01 mole) of (E)-1-(4-chloropheny1)-2-(1,2,4-triazol-1-yl)-4,4-dimethyl-1-penten-3-one(melting point 108 - 109°C) characterized by the NMR spectrum given below. To the solution was added 0.38 g (0.01 mole) of sodium borohydride, while keeping the temperature of the reaction system at 20°C or below by cooling in ice. To the solution, after having been kept at 20°C for 3 hours, was added 1 ml of acetic acid to effect decomposition. The organic layer was extracted with 100 ml of ethyl acetate and the extract was washed with 50 ml of a 5% aqueous nydrogencarbonate solution, and dried over anhydrous sodium sulfate. After the removal of the solvent by distillation under reduced pressure, the residue was recrystallized from n-hexane to obtain 2.0 g (69% yield) of the captioned compound having a melting point of 153 - 155°C. The elementary analysis and NMR spectrum (determined on a solution in deuterochloroform and expressed in terms of δ value) of each compound were as shown below:

(E)-1-(4-chlorophenyl)-2-(1,2,4-triazol-1-yl)-4,4-dimethyl-1-penten-3-one:

| Elementary analysis: | | | | |
|---|---|---|---|---|
| | C% | H% | N% | Cl% |
| calculated for $C_{15}H_{16}N_3OCl$ | 62.17 | 5.58 | 14.50 | 12.23 |
| Found | 62.32 | 5.60 | 14.41 | 12.20 |

NMR spectrum:

8.11 (1H, singlet, triazole proton), 7.90 (1H, singlet, triazol proton), 7.15 (4H, singlet, phenyl proton), 6.99 (1H, singlet, olefin proton), 0.99 (9H, singlet, butyl proton).

(E)-1-(4-chlorophenyl)-2-(1,2,4-triazol-1-yl)-4,4-dimethyl-1-penten-3-ol:

| Elementary analysis: | | | | |
|---|---|---|---|---|
| | C% | H% | N% | Cl% |
| Calculated for $C_{15}H_{18}N_3OCl$ | 61.74 | 6.23 | 14.40 | 12.15 |
| Found | 61.82 | 6.38 | 14.38 | 12.15 |

NMR spectrum:

8.52 (1H, singlet, triazole proton), 7.98 (1H, singlet, triazole proton), 7.30 (4H, singlet, phenyl proton), 6.91 (1H, singlet, olefin proton), 4.56 (2H, broad singlet, hydroxyl proton and proton of methyne group bearing hydroxyl group), 0.66 (9H, Singlet, butyl proton).

Example 2

Synthesis of (+)-(E)-1-(4-chlorophenyl)-2-(1,2,4-triazol-1-yl)-4,4-dimethyl-1-penten-3-ol by asymmetric reduction:

Into 40 cc of an ethyl ether solution containing 1.25 g (0.033 mole) of $LiAlH_4$, while being cooled in ice, was added dropwise over a period of 30 minutes 100 cc of an ethyl ether solution containing 6.1 g (0.034 mole) of (-)-N-methylephedrine, and the mixture was then stirred at room temperature for 15 minutes. To the mixture was added dropwise over a period of 30 minutes 45 cc of an ethyl ether solution containing 8.24 g (0.068 mole) of N-ethylaniline, and the resulting mixture was stirred at room temperature for 3 hours. To the mixture was further added 60 cc of an ethyl ether solution containing 2.9 g (0.01 mole) of (E)-1-(4-chlorophenyl)-2-(1,2,4-triazol-1-yl)-4,4-dimethyl-1-penten-3-one over a period of 12 minutes at -70° to -67°C, and the mixture was stirred at -73°C for 3 hours under adiabatic conditions. The mixture was left standing overnight at room temperature, and to the mixture was added 110 cc of 2N hydrochloric acid to effect decomposition. The separated organic layer was washed with 100 cc of a saturated aqueous sodium hydrogencarbonate solution, then with 100 cc of ice water, dried over anhydrous sodium sulfate and concentrated under

reduced pressure to obtain 3.0 g of crystals of a triazolyl alcohol compound: $[\alpha]_D^{24}$ + 9.0 (c = 1.0, $CHCl_3$). A 2.5 g portion of the obtained crystals was recrystallized twice from a cyclohexane-dioxane mixture to obtain 0.81 g of (+)-(E)-1-(4-chlorophenyl)-2-(1,2,4-triazol-1-yl)-4,4-dimethyl-1-penten-3-ol: $[\alpha]_D^{24}$ + 15.7 (c = 1.0, $CHCl_3$); melting point 169 - 170°C. The NMR spectrum was identical with that of the racemate shown in Reference Example 1.

Example 3

Asymmetric reduction by use of (-)-menthol:

To 0.4 g (0.01 mole) of $LiAlH_4$ dissolved in 30 cc of THF, was added at 10°C 3Occ of a THF solution containing 4.4 g (0.028 mole) of (-)-menthol. The mixture was then stirred at room temperature for 30 minutes. To the mixture was added at -30°C 50 cc of a THF solution containing 2.0 g (0.007 mole) of 1-(4-chlorophenyl)-2-(1,2,4-triazol-1-yl) -4,4-dimethyl-1-penten-3-one. The mixture was then stirred for 2 hours while keeping the temperature at -5°C. After adding to the mixture 5 cc of 1N hydrochloric acid and removing the insolubles by filtration, the filtrate was poured into 300 cc of ice water and extracted with 500 cc of ethyl ether. The organic layer was washed with 200 cc of a saturated sodium hydrogencarbonate solution, then with 200 cc of ice-cooled water, dried over anhydrous sodium sulfate, and concentrated under reduced pressure to obtain a crude oily product. The crude product was fractionally purified by Silica gel column chromatogrephy (100 g silica gel; developing solvent: n-hexane/acetone = 30/l). There was obtained 0.5 g of unreacted ketone compound which was recovered and 1.3 g of crystals of (+)-(E)-1-(4-chlorophenyl)-2-(1,2,4-triazol-1-yl)4,4-dimethyl-1-penten-3-ol (by crystallization from a carbon tetrachloride-n-hexane mixture): $[\alpha]_D^{26}$ + 5.0 (c = 1, $CHCl_3$).

Example 4

Asymmetric reduction by use of (-)-borneol.

To 0.2 g (0.0053 mole) of $LiAlH_4$ dissolved in 30 cc of THF, was added at 0°C 30 cc of a THF solution containing 2.4 g (0.0155 mole) of (+)-borneol. The mixture was then stirred at room temperature for 50 minutes. To the mixture was added at 0°C 30 cc of a THF solution containing 1.0 g (0.0034 mole) of (E)-1-(4-chlorophenyl)-2-(1,2,4-triazol-1-yl) -4,4-dimethyl-1-penten-3-one. The mixture was then stirred at room temperature for 3 hours. After adding 0.5 cc of 1N hydrochloric acid to the mixture and removing the insolubles by filtration, the filtrate was poured into 300 cc of ice-cooled water, and extracted with 500 cc of ethyl ether. The organic layer was washed with 200 cc of a saturated aqueous sodium hydrogencarbonate solution, then with 200 cc of ice-cooled water, dried over anhydrous sodium sulfate, and concentrated under reduced pressure to obtain a crude oily substance. The crude substance was fractionally purified by silica gel column chromatography (100 g silica gel; developing solvent: n-hexane/acetone = 30/l). There were obtained 0.4 g of the unreacted ketone which was recovered and 0.45 g of crystals of (+)-(E)-1-(4-chlorophenyl)-2-(1,2,4-triazol-1-yl) -4,4-dimethyl-1-penten-3-ol (by crystallization from a carbon tetrachloride-n-hexane mixture): $[\alpha]_D^{24}$ + 3.2 (c = 1, $CHCl_3$).

The useful properties of the (+)-triazolyl alcohol derivative of this invention are illustrated below in detail with reference to some examples of tests performed on (+)-(E)-(4-chlorophenyl)-2-(1,2,4-triazol1-yl)-4,4-dimethyl-1-penten-3-ol (compound No. 1) obtained in Example 1 using as reference samples corresponding (-)-triazolyl alcohol derivative obtained in Example 1 (compound No.2) and the racemate obtained in Reference Example 1 (compound No. 3).

Test Example 1

Inhibition of fungus growth:

A medium containing 5 g of polypeptone, 20 g of malt extract, 20 g of sucrose and 20 g of agar per liter of water heated to form a liquid. To the liquefied medium, was added a predetermined quantity of a diluted sample of the test compound in the form of emulsifiable concentrate so as to keep the concentration of each sample in the medium at a predetermined level. After thorough stirring, the medium was poured into a Petri dish to form an agar plate. After the agar had set, it was inoculated with a colony or a conidium suspention of the test fungus. The fungus species and the incubation period (the period of time from inoculation to observation) were as shown below. The incubation temperature was 20°C for Venturia inaequalis and 28°C for other fungi.

| Fungus species | Abbreviation | Incubation period (day) |
|---|---|---|
| Helminthosporium gramineum | Hg | 6 |
| Penicillium italicum | Pi | 6 |
| Venturia inaequalis | Vi | 7 |
| Valsa mali | Vm | 4 |
| Mycosphaerella melonis | Mm | 4 |
| Diaporthe citri | Dc | 6 |
| Ustilago nuda | Un | 6 |
| Verticillium albo-atrum | Va | 7 |
| Septoria tritici | St | 7 |
| Cercospora beticola | Cb | 7 |

The fungus growth inhibitory activity of the test compound was evaluated by the concentration which inhibits 90% of the mycelium growth ($ED_{90}$). As is seen from the results shown in Table 1, it was found that the triazolyl alcohol derivative of this invention (compound No. 1) show markedly lower antifungal activity compared with the corresponding (-)-triazolyl alcohol derivative (compounds No. 2) and the racemate (compound No. 3).

Further test data showing the low antifungal activity of the present compound can be seen in EP-A-0054431.

Table 1. Concentration, in ppm, which inhibits 90% colony growth ($ED_{90}$).

| Fungus species | Compound | | |
|---|---|---|---|
| | No.2 | No.1 | No.3 |
| Hg | 1.5 | > 25.0 | 6.0 |
| Pl | 0.1 | > 25.0 | 0.4 |
| Vl | < 0.1 | 25.0 | 0.1 |
| Vm | 0.1 | > 25.0 | 0.4 |
| Mn | 0.3 | > 25.0 | 1.3 |
| Dc | 0.4 | > 25.0 | 5.0 |
| Un | 5.0 | > 25.0 | 5.0 |
| Va | 0.4 | > 25.0 | 1.3 |
| St | 0.4 | > 25.0 | 1.0 |
| Cb | 0.4 | > 25.0 | 1.5 |

The results of examination on the plant growth regulatory activity and the herbicidal activity are given below. It is shown that the said activities of (+)-triazolyl alcohol derivatives and racemates are far higher than those of (-)-triazolyl alcohol derivatives.

Test Example 2

Test on wheat for growth retardation:

A 85-ml plastic pot filled with sandy loam was seesed with 10 to 15 seeds of wheat (var. Chikugo No. 2), which had been soaked in an aqueous diluted liquor of the test compound in emulsifiable concentrate form, and cultivated at a controlled temperature of 18° to 23°C for 7 days. The leaf length was then measured and the percentage elongation in the control plot.

$$\text{Elongation (\%)} = \frac{\text{Plant length in treated plot}}{\text{Plant length in control plot}} \times 100$$

The test results were as shown in Table 2. It was found that the plant growth retarding activity of the (+)-triazolyl alcohol derivative is far higher than that of the (-)-triazolyl alcohol derivative or a racemate.

Table 2

| Compound No. | Concentration of treating liquor (ppm) | Plant length (mm) | Elongation (%) |
|---|---|---|---|
| 2. | 12.5 | 95 | 69 |
| | 3.1 | 115 | 83 |
| 1 | 12.5 | 45 | 33 |
| | 3.1 | 61 | 44 |
| 3 | 12.5 | 62 | 45 |
| | 3.1 | 83 | 60 |
| Control (untreated) | - | 138 | 100 |

Test Example 3

Dwarfing test on pot-mum:

Pot-mum (var. Paragon) was cultivated in a 4.8-inch clay pot filled with 500 g of an artificial soil composed of sea sand, mountain soil and peat. Two weeks after setting, the plant was pinched so as to undergo the triple stem training. Two weeks after pinching, when sprouting had already begun, the test compound diluted to a predetermined concentration was applied to the plant and the growth retarding effect was inspected 42 days after the treatment. The results were as shown in Table 3

The effect was evaluated in the following way: the difference between the plant height at the time of application of the chemical and the plant height on the 42nd day after the application is recorded and expressed as elongation index which is the percentage of said difference based on the similar difference in the untreated plot. The indices shown in Table 3 were mean values of three replications.

All of the test compounds showed inhibition of internode elongation and reduction in plant height, but the phytotoxicity such as necrosis or chlorisis was not observed and even the green color of leaves became deeper. The (+)-triazolyl alcohol derivative (compound No. 1) showed a far stronger dwarfing effect compared with the (-)-triazolyl alcohol derivatives (compound No. 2) and a stronger effect compared with the racemate (compound No. 3).

Table 3.

| Dwarfing test on pot-mum. | | |
|---|---|---|
| Compound No. | Concentration of treating solution (ppm) | Elongation index (%) |
| 1 | 200 | 15 |
| | 100 | 23 |
| | 50 | 39 |
| 2 | 200 | 88 |
| | 100 | 94 |
| | 50 | 103 |

Continuation of the Table on the next page

Table 3. (continued)

| Dwarfing test on pot-mum. | | |
|---|---|---|
| Compound No. | Concentration of treating solution (ppm) | Elongation index (%) |
| 3 | 200 | 21 |
| | 100 | 42 |
| | 50 | 78 |

Test Example 4

Test on apple seedling for growth retardation of current shoot:

An apple seedling (var. Golden Delicious) planted in a 18-cm clay pot was pruned and cultivated in a greenhouse. Three weeks after the emrgence of current shoots, the above-ground part of the plant was entirely treated with a liquid preparation of the test compound in predetermined concentration by means of a hand-sprayer. Fourteen days after the treatment, the lengths of current shoots were measured and the amount of elongation was obtained from the difference between said length and the length at the time of chemical treatment. Two pots of the plant were used for each chemical treatment and the growth length determined on 4 to 6 shoots. The results in average value were as shown in Table 4. The (+)-triazolyl alcohol derivative showed a far higher growth inhibitory activity as compared with the (-)-triazolyl alcohol derivative and racemate.

Table 4

| Test on apple tree for growth retardation of current shoot. | | | |
|---|---|---|---|
| Compound No. | Concentration of treating liquor (ppm) | Elongation | |
| | | (mm) | (%) |
| 1 | 100 | 33 | 18 |
| | 50 | 64 | 36 |
| | 25 | 79 | 44 |
| 2 | 100 | 189 | 106 |
| | 50 | 168 | 94 |
| | 25 | 185 | 103 |
| 3 | 100 | 65 | 36 |
| | 50 | 78 | 44 |
| | 25 | 93 | 52 |
| Control (untreated) | - | 179 | 100 |

Test Example 5.

Test on lawn grass for growth retardation:

A 1/5,000 - are Wagner pot filled with upland soil was seeded with seeds of lawn grass (var. Seaside grass). After covering with soil, the seeds were cultivated in a greenhouse. After one month, the grass was cut at a height of 1 cm from the ground level and both foliage and soil were treated with a predetermiend amount of the chemical preparation by using a hand sprayer. Two weeks after the treatment, the elongation of grass was examined, then the grass was cut again and the cultivation was continued for additional 4 weeks. The results of examination performed after two weeks (first examination) and four weeks (second examination) from the treatment were as shown in Table 5. As compared with the (-)triazolyl alcohol derivative and racemate, the (+)-triazolyl alcohol derivative showed a far greater effect.

Table 5.

| Test on lawn grass for growth retardation. | | | | |
|---|---|---|---|---|
| Compound No. | Application rate (g/are) | Growth of lawn grass (cm) | | |
| | | 1st time | 2nd time | Total |
| 1 | 10 | 1.0 | 0.5 | 1.5 |
| | 5 | 1.3 | 0.5 | 1.8 |
| | 2.5 | 1.5 | 1.0 | 2.5 |
| 2 | 10 | 4.3 | 8.0 | 12.3 |
| | 5 | 4.5 | 8.5 | 13.0 |
| | 2.5 | 5.0 | 9.0 | 14.0 |
| 3 | 10 | 1.5 | 1.0 | 2.5 |
| | 5 | 1.8 | 2.0 | 3.8 |
| | 2.5 | 2.0 | 3.0 | 5.0 |
| Untreated | - | 5.0 | 9.0 | 14.0 |

Test Example 6.

Pot test of naked barley:

A 1/2,000 - are Wagner pot was filled with paddy soil of the plough layer, which passed through a wire screen having a square aperture of 1.5 x 1.5 cm. After applying as basal fertilizer a urea-base compound fertilizer at an application rate of $N/P_2O_5/K_2O$ = 1.3/1.3/1.3 g/pot, the pot was seeded with 12 seeds of naked barley (var. Hinodehadaka) on December 5. The seeds were cultivated in a greenhouse. When the seedling had emerged and grown to a height of several centimeters, the seedlings were thinned to 6 stumps per pot. At the beginning of internode elongation (Feb. 15), a predetermiend amount of the chemical preparation was sprayed over the soil surface and the cultivation was further continued until the harvest time (May 21) had reached when the plant height, number of ear, and the weight of husked barley were measured. The results of examination were as shown in Table 6. Although all of the test compounds showed a dwarfing, tiller-promoting, and yield increasing effect, the (+)-triazolyl alcohol derivative exhibited much superior effect compared with the (-)-triazolyl alcohol derivative and a stronger dwarfing effect compared with the racemate.

Table 6. **Pot test of naked barley.**

| Compound No. | Application rate, active ingredient (g/are) | Number of ears (per pot) | Plant height (cm) | Weight of husked barley (g/pot) | Weight of husked barley (%) |
|---|---|---|---|---|---|
| 1 | 1<br>5 | 58.0<br>60.0 | 64.7<br>50.4 | 71.7<br>66.2 | 118<br>109 |
| 2 | 1<br>5 | 46.7<br>48.7 | 88.7<br>75.3 | 60.0<br>61.2 | 99<br>101 |
| 3 | 1<br>5 | 57.7<br>60.0 | 76.4<br>54.3 | 74.3<br>67.3 | 123<br>111 |
| Untreated | - | 45.3 | 85.9 | 60.6 | 100 |

Test Example 7.

Weed control test in upland soil:

A 1/1,000 - are Wagner pot was filled with a soil mixed with seeds of large crabgrass, green amaranth, and fat hen. A diluted aqueous emulsion containing a prescribed quantity of the test compound was applied by means of a hand sprayer to treat the soil surface. After the treatment, sugar beet seedlings (var. Monohill) in fifth leaf age bred in a paper pot were transplanted to the Wagner pot and bred in a greenhouse. The weed control activity and phytotoxicity of the test compound were observed on 20th day from the treatment. The results of observation were as shown in Table 7.

The evaluation of weed control activity was performed by classifying the observed results into the following 6 grades of from 0 to 5.

The phytotoxicity was evaluated likewise.

| | Degree of weed control (%) |
|---|---|
| 0 | 0 - 9 |
| 1 | 10 - 29 |
| 2 | 30 - 49 |
| 3 | 50 - 69 |
| 4 | 70 - 89 |
| 5 | 90 - 100 |

Table 7. **Weed control test in upland soil.**

| Compound No. | Application rate (g/are) | Weed control activity | | | Phytotoxicity to sugar beet |
|---|---|---|---|---|---|
| | | Large crabgrass | Green amaranth | Fat hen | |
| 1 | 20 | 5 | 5 | 5 | 0 |
| | 10 | 5 | 5 | 5 | 0 |
| 2 | 20 | 4 | 4 | 5 | 0 |
| | 10 | 3 | 3 | 4 | 0 |
| 3 | 20 | 5 | 5 | 5 | 0 |
| | 10 | 4 | 5 | 5 | 0 |

Preparation Example 1 Dust.

Two parts of the compound, 88 parts of clay and 10 parts of talc are thoroughly mixed by grinding to form a dust preparation containing 2% of the active ingredient.

Preparation Example 2 Wettable powder.

Thirty parts of the compound, 45 parts of diatomaceous earth, 20 parts of white carbon, 3 parts of a wetting agent (sodium lauryl sulfate) and 2 parts of a dispersant (calcium ligninsulfonate) are thoroughly mixed by grinding to form a wettable powder preparation containing 30% of the active ingredient.

Preparation Example 3 Emulsifiable concentrate.

Ten parts of the compound, 80 parts of cyclohexanone and 10 parts of an emulsfier (polyoxyethylene alkylaryl ether) are mixed to form an emulsifiable concentrate containing 10% of the active ingredient.

Preparation Example 4 Liquid.

0.05 Parts by weight of the compound, 1 part by weight of "Hymal 1009" (a surfactant produced by Matsumoto Yushi Co.), 1 part by weight of "Newcol 560" (a nonionic emulsifier), 2.5 parts by weight of cyclohexanone and 95.45 parts by weight of water are mixed to form a liquid preparation.

## Claims

1. The triazolyl alcohol derivative of formula (I)

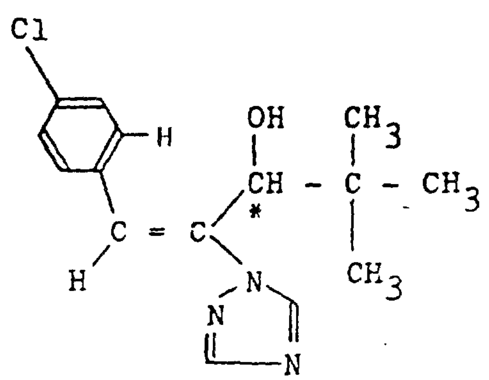

(I)

wherein the asterisk indicates an asymmetric carbon atom, having an optical activity of (+) and the E geometric configuration, and salts thereof.

2. A process for producing the triazolyl alcohol derivative of claim 1, which process comprises asymmetrically reducing a triazolyl ketone derivative of formula (II):

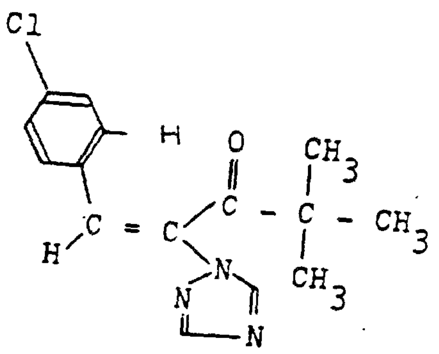

(II)

(II)

with a chiral reducing agent of the modified lithium aluminium hydride type formed by the partial decomposition of lithium aluminium hydride with an optically active alcohol.

3. A process according to claim 2, wherein use is made of the chiral reducing agent of the modified lithium aluminium hydride type prepared from 1 equivalent of lithium aluminium hydride, 1 equivalent of the (-) optical isomer of an N-substituted ephedrine and 2 equivalents of an N-alkylaniline.

4. A process according to claim 2, wherein use is made of the chiral reducing agent of the modified lithium aluminium hydride type prepared from 1 equivalent of lithium aluminium hydride, 1 equivalent of the (-) optical isomer of a 2-N,N-disubstituted amino-1-phenylethanol, and 2 equivalents of an N-alkylaniline.

5. A process according to claim 2, wherein use is made of the chiral reducing agent of the modified lithium aluminium hydride type prepared from 1 equivalent of lithium aluminium hydride and 2 to 3 equivalents of (-) menthol.

6. A process for producing the triazolyl alcohol derivative defined in claim 1, which process comprises reacting a racemate of a triazolyl alcohol derivative of formula (Ia):

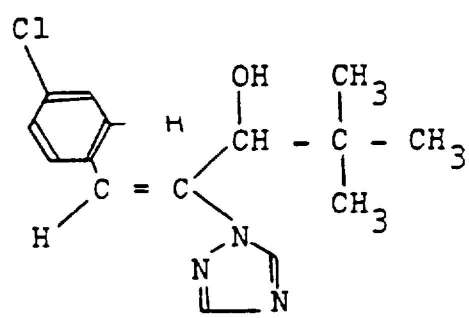

(Ia)

with a reactive derivative of an optically active carboxylic acid to form two diastereomeric esters, isolating diastereomeric ester of the (+)-triazolyl alcohol and hydrolyzing the isolated ester.

7. A process according to claim 6, wherein (+)-menthoxylacetyl chloride is used as the reactive derivative of an optically active carboxylic acid.

8. A plant growth regulatory and herbicidal composition containing a plantphysiologically acceptable inert carrier and, as an active ingredient, the triazolyl alcohol derivative of formula (I):

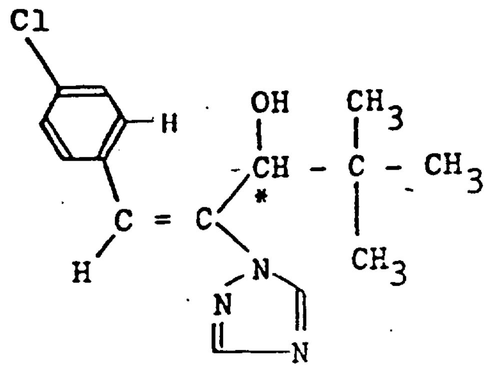

(I)

wherein the asterisk indicates an asymmetric carbon atom, having an optical activity of (+) and the E geometric configuration.

**9.** A method for controlling plant growth, which comprises applying a plant growth regulatory composition according to claim 8 to the plant.

**10.** A method for killing weeds, which comprises applying a herbicidal composition according to claim 8 to the weeds.

**11.** The use of a triazolyl alcohol derivative of formula (I):

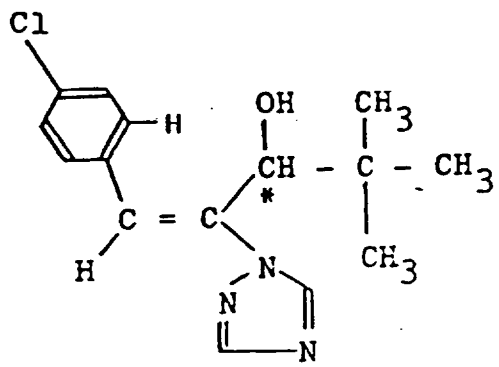

(I)

wherein the asterisk indicates an asymmetric carbon atom having an optical activity of (+) and the E-geometric configuration in the manufacture of a plant growth regulatory preparation for treatment of plants.

**12.** The use a triazolyl alcohol derivative of formula (I):

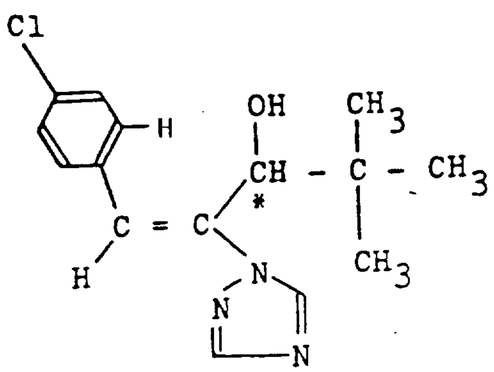

(I)

wherein the asterisk indicates an asymmetric carbon atom, having an optical activity of (+) and the E-geometric configuration in the manufacture of a herbicidal preparation for treatment of weeds.

## Patentansprüche

1. Triazolylalkoholderivat der Formel (I)

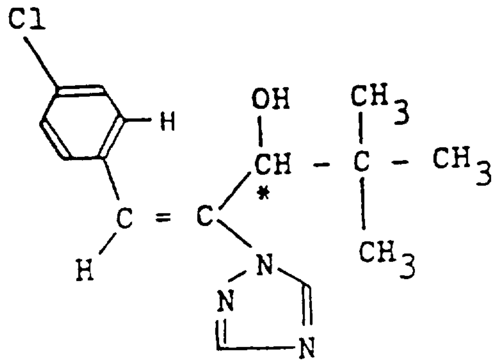

(I)

in der der Stern ein asymmetrisches Kohlenstoffatom bezeichnet, mit einer optischen Aktivität von (+) und einer geometrischen E-Konfiguration und die Salze davon.

2. Verfahren zur Herstellung des Triazolylalkoholderivats nach Anspruch 1, wobei das Verfahren die asymmetrische Reduktion eines Triazolylketonderivats der Formel (II):

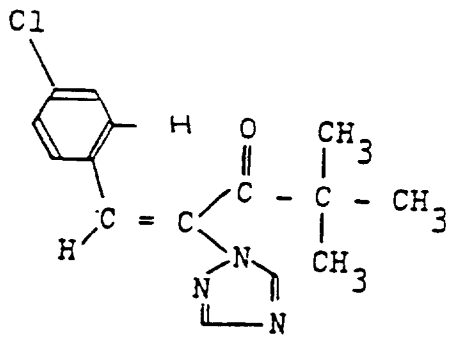

(II)

(II)

mit einem chiralen Reduktionsmittel des modifizierten Lithiumaluminiumhydridtyps umfaßt, das durch teilweise Zersetzung von Lithiumaluminiumhydrid mit einem optisch aktiven Alkohol gebildet wurde.

3. Verfahren nach Anspruch 2, wobei das chirale Reduktionsmittel des modifizierten Lithiumaluminiumhydridtyps verwendet wird, das aus 1 Äquivalent Lithiumaluminiumhydrid, 1 Äquivalent des optischen (-)-Isomers eines N-substituierten Ephedrins und 2 Äquivalenten eines N-Alkylanilins hergestellt wurde.

4. Verfahren nach Anspruch 2, wobei das chirale Reduktionsmittel des modifizierten Lithiumaluminiumhydridtyps verwendet wird, das aus 1 Äquivalent Lithiumaluminiumhydrid, 1 Äquivalent des optischen (-)-Isomers eines 2-N,N-disubstituierten Amino-1-phenylethanols und 2 Äquivalenten eines N-Alkylanilins hergestellt wurde.

5. Verfahren nach Anspruch 2, wobei das chirale Reduktionsmittel des modifizierten Lithiumaluminiumhydridtyps verwendet wird, das aus 1 Äquivalent Lithiumaluminiumhydrid und 2 bis 3 Äquivalenten (-)-Menthol hergestellt wurde.

6. Verfahren zur Herstellung des Triazolylalkoholderivats nach Anspruch 1, wobei das Verfahren die Umsetzung eines Racemats eines Triazolylalkoholderivats der Formel (Ia):

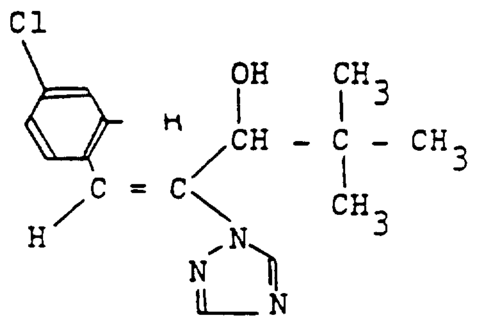

(Ia)

mit einem reaktiven Derivat einer optisch aktiven Carbonsäure, wobei zwei diastereomere Ester gebildet werden, Isolieren des diastereomeren Esters des (+)-Triazolylalkohols und Hydrolysieren des isolierten Esters umfaßt.

**7.** Verfahren nach Anspruch 6, wobei (+)-Menthoxylacetylchlorid als reaktives Derivat einer optisch aktiven Carbonsäure verwendet wird.

**8.** Pflanzenwachstumsregulierendes und herbizides Mittel, das einen pflanzenphysiologisch verträglichen inerten Träger und als Wirkstoff das Triazolylalkoholderivat der Formel (I) enthält:

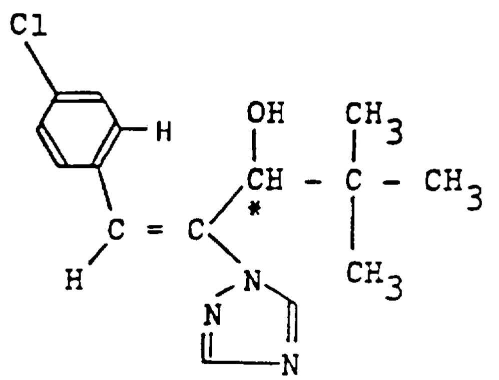

(I)

in der der Stern ein asymmetrisches Kohlenstoffatom bezeichnet, mit einer optischen Aktivität von (+) und einer geometrischen E-Konfiguration.

**9.** Verfahren zur Regelung des Pflanzenwachstums, umfassend das Aufbringen eines pflanzenwachstumsregulierenden Mittels nach Anspruch 8 auf die Pflanze.

**10.** Verfahren zur Abtötung von Unkräutern, umfassend das Aufbringen eines herbiziden Mittels nach Anspruch 8 auf die Unkräuter.

**11.** Verwendung eines Triazolylalkoholderivats der Formel (I):

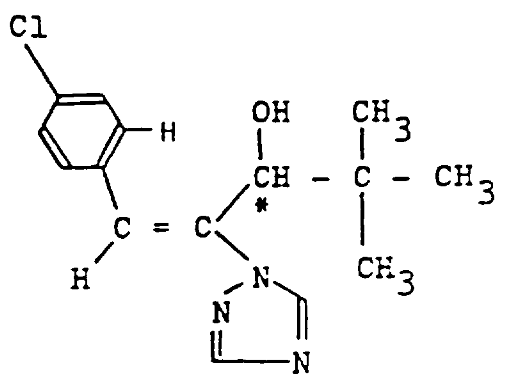

(I)

in der der Stern ein asymmetrisches Kohlenstoffatom bezeichnet, mit einer optischen Aktivität von (+) und einer geometrischen E-Konfiguration, zur Herstellung einer pflanzenwachstumsregulierenden Zubereitung zur Behandlung von Pflanzen.

**12.** Verwendung eines Triazolylalkoholderivats der Formel (I):

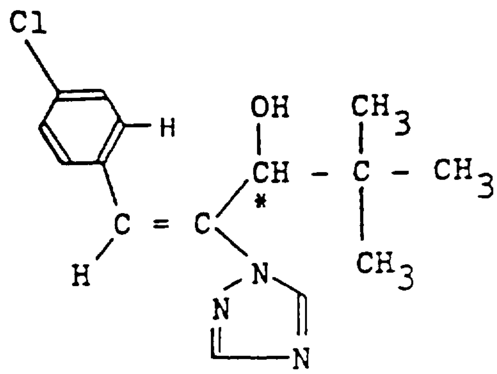

(I)

in der der Stern ein asymmetrisches Kohlenstoffatom bezeichnet, mit einer optischen Aktivität von (+) und einer geometrischen E-Konfiguration, zur Herstellung einer herbiziden Zubereitung zur Behandlung von Unkräutern.

**Revendications**

**1.** Dérivé d'alcool triazolylique de formule (I)

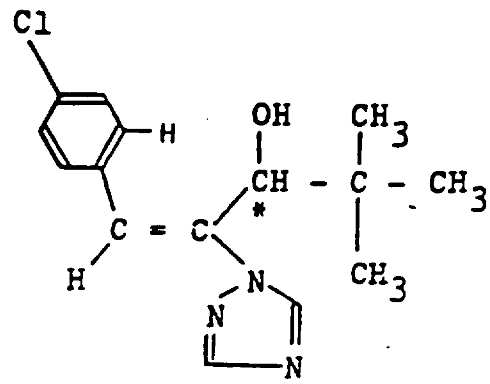

(I)

dans laquelle l'astérisque indique un atome de carbone asymétrique, ayant une activité optique (+) et la configuration géométrique E, et sels de ce dérivé.

**2.** Procédé de préparation du dérivé d'alcool triazolylique selon la revendication 1, consistant à réduire asymétriqement un dérivé de triazolylcétone de formule (II)

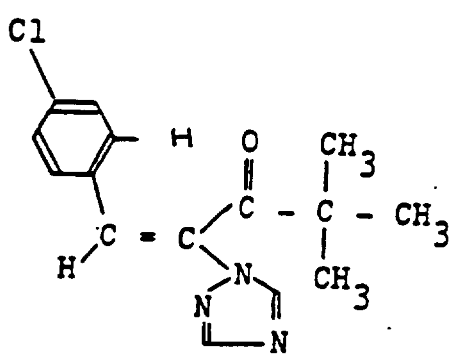

(II)

(II)

avec un agent réducteur chiral de type hydrure de lithium et d'aluminium modifié, formé par décomposition partielle

d'hydrure de lithium et d'aluminium par un alcool optiquement actif.

3. Procédé selon la revendication 2, dans lequel on utilise l'agent réducteur chiral de type hydrure de lithium et d'aluminium modifié, préparé à partir de 1 équivalent d'hydrure de lithium et d'aluminium, de 1 équivalent de l'isomère optique (-) d'une éphédrine N-substituée et de 2 équivalents d'une N-alkylaniline.

4. Procédé selon la revendication 2, dans lequel on utilise l'agent réducteur chiral de type hydrure de lithium et d'aluminium modifié, préparé à partir de 1 équivalent d'hydrure de lithium et d'aluminium, de 1 équivalent de l'isomère optique (-) d'un amino-1-phényléthanol 2-N,N-bisubstitué et de 2 équivalents d'une N-alkylaniline.

5. Procédé selon la revendication 2, dans lequel on utilise l'agent réducteur chiral de type hydrure de lithium et d'aluminium modifié, préparé à partir de 1 équivalent d'hydrure de lithium et d'aluminium et de 2 à 3 équivalents de (-) menthol.

6. Procédé de préparation du dérivé d'alcool triazolylique selon la revendication 1, consistant à faire réagir un racémate d'un dérivé d'alcool triazolylique de formule (Ia)

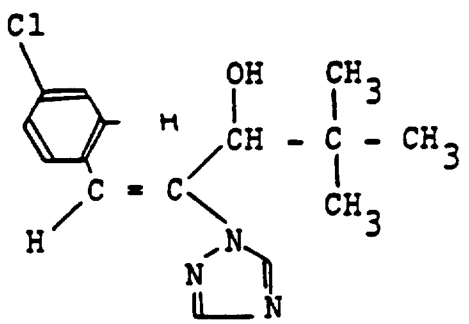

(Ia)

avec un dérivé réactif d'un acide carboxylique optiquement actif pour former deux esters diastéréoisomères, à isoler l'ester diastéréoisomère de l'alcool (+)-triazolylique et à hydrolyser l'ester isolé.

7. Procédé selon la revendication 6, dans lequel le chlorure de (+)-menthoxyacétyle est utilisé comme dérivé réactif d'un acide carboxylique optiquement actif.

8. Composition régulatrice de la croissance des plantes et herbicide, contenant un véhicule inerte acceptable du point de vue de la physiologie vegétale et, en tant qu'ingrédient actif, le dérivé d'alcool triazolylique de formule (I)

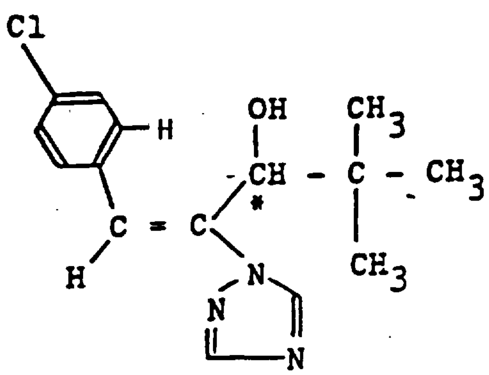

(I)

dans laquelle l'astérisque indique un atome de carbone asymétrique, ayant une activité optique (+) et la configuration géométrique E.

9. Procédé de régulation de la croissance des plantes, consistant à appliquer sur les plantes une composition régulatrice de la croissance des plantes selon la revendication 8.

10. Procédé de destruction des mauvaises herbes, consistant à appliquer sur les mauvaises herbes une composition herbicide selon la revendication 8.

**11.** Utilisation d'un dérivé d'alcool triazolylique de formule (I)

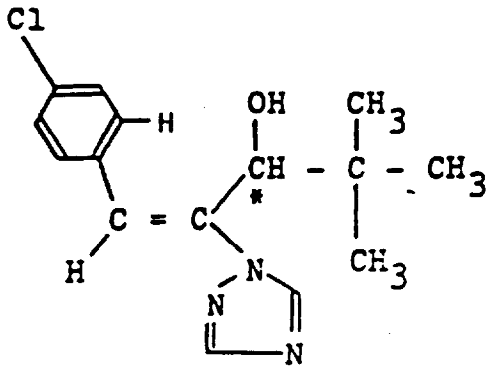

(I)

dans laquelle l'astérisque indique un atome de carbone asymetrique, ayant une activité optique (+) et la configuration geometrique E, dans la préparation d'une composition régulatrice de la croissance pour le traitement de plantes.

**12.** Utilisation d'un dérivé d'alcool triazolylique de formule (I)

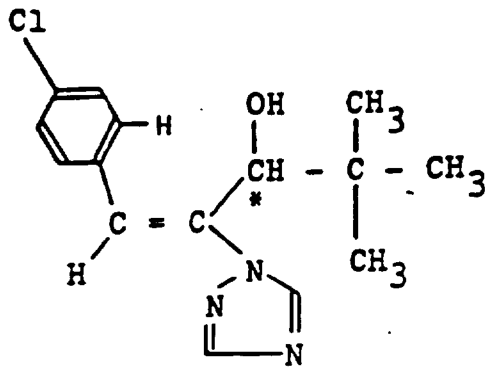

(I)

dans laquelle l'astérisque indique un atome de carbone asymétrique, ayant une activité optique (+) et la configuration géométrique E, dans la préparation d'une composition herbicide pour le traitement de mauvaises herbes.